# EUROPÄISCHE PATENTSCHRIFT

(11) **EP 0 781 325 B1**
(45) Veröffentlichungstag und Bekanntmachung des Hinweises auf die Patenterteilung: **12.01.2000**
(21) Anmeldenummer: 95933363.4
(22) Anmeldetag: 12.09.1995
(51) Int. Cl.: C12M 1/26, G01N 1/10

(54) **NEUES PROBENAHMESYSTEM FÜR DIE BIOPROZESS ANALYTIK**
NOVEL SAMPLING SYSTEM FOR USE IN THE ANALYSIS OF BIOLOGICAL PROCESSES
NOUVEAU SYSTEME DE PRELEVEMENT D'ECHANTILLONS POUR L'ANALYSE DE PROCESSUS BIOLOGIQUES

(30) Priorität: 13.09.1994 DE 4432599
(43) Veröffentlichungstag der Anmeldung: 02.07.1997
(73) Patentinhaber: Boehringer Ingelheim Pharma KG, 55216 Ingelheim am Rhein (DE)
(72) Erfinder: SCHORN, Peter, D-89129 Langenau (DE); VOIGT, Hans, D-88400 Biberach (DE); NOE, Wolfgang, D-88400 Biberach (DE)
(74) Vertreter: Laudien, Dieter, Dr.
(86) Internationale Anmeldenummer: EP9503587
(87) Internationale Veröffentlichungsnummer: WO9608556

(56) Entgegenhaltungen:
- WO-A-88/00234
- DE-B- 2 659 923
- US-A- 4 942 770

## Beschreibung

Gegenstand der vorliegenden Erfindung ist eine neuartige Probenahmevorrichtung, die eine sterile Entnahme von Zellkulturproben und sonstigen flüssigen Medien aus Bioreaktoren oder Sterilbehältern ermöglicht, wobei der Schutz vor Rückkontamination mit Hilfe eines sterilfiltrierten inerten Gasstromes gewährleistet wird.

Die Optimierung biotechnologisch relevanter Herstellverfahren hängt in erster Linie von Fortschritten im Bereich der Bioprozeßanalytik ab, weil hieraus Erkenntnisse für eine verbesserte Prozeßführung gewonnen werden können. In zunehmendem Maße werden hierzu moderne Analyseverfahren wie Zytofluorometrie, Hochdruck-Flüssigkeits-Chromatographie und Biosensoren eingesetzt. Voraussetzung dafür ist die Anwendung geeigneter Probenahmesysteme, welche das Verbindungsglied zum Fermenterinhalt darstellen.

Bei der Kultivierung von tierischen, bakteriellen und pflanzlichen Zellen kommt der aseptischen Betriebsweise und den steriltechnischen Komponenten der Fermentationen besondere Bedeutung zu, da während des Fermentationsprozesses laufend Proben gezogen werden müssen, um wichtige Parameter wie Wachstumsverhalten der Kultur, Zellzustandsgrößen und Stoffwechselwerte bestimmen zu können.

Die eigentliche Probenahme am Bioreaktor stellt einen kritischen Vorgang dar, weil damit die Sterilbarriere zum Fermenterinhalt durchbrochen wird. Daher ist bei mehrere Wochen dauernden Fermentationen beziehungsweise hoher Probenahmefrequenz eine einfache und zuverlässige Probenahmemöglichkeit von großer Bedeutung für den Prozeßerfolg.

Weiterhin muß eine Probenahme den Fermenterinhalt repräsentativ wiedergeben und ständig verfügbar sein, wobei der Einsatz automatisierter Analysensysteme zunehmend die Möglichkeit einer vollautomatischen Probenziehung verlangt.

Die bisherigen Probenahmevorrichtungen für diesen Zweck werden entweder von Herstellern von Bioreaktoren angeboten oder stellen Eigenkonstruktionen des Anwenders dar. Diese oft improvisierten Hilfsmittel können die genannten Forderungen nur teilweise erfüllen oder sind mit folgenden spezifischen Nachteilen behaftet:

Ein wichtiges Konstruktionsmerkmal ist hierbei die Art der Vermeidung von Rückkontaminationen des Fermenterinhaltes über den Probenahmeweg, da dies die Schnittstelle zur mikrobiologisch belasteten Umgebung darstellt.

Diese Aufgabe wird auf unterschiedliche Weise gelöst:
- Durchstechen von Septen oder Membranen auf vorsterilisierten Probenfläschchen. Diese Methode wird häufig, vor allem in der Bakterienfermentation, angewandt. Diese Methode ist aber umständlich und wenig sicher zu handhaben und erfordert die chemische oder thermische Desinfektion der Anstechnadel, z. B. mittels Abflammen.
- Desinfektion eines Probenahmeweges durch chemische Agenzien wie z.B. Ethanol. Diese Methode bringt die Gefahr der Probenbeeinflussung, den unerwünschten Anfall von Chemikalien und einen risikoreichen Neuanschluß des Probengefäßes mit sich.
- Verwendung geschlossener Probenahmesysteme mit Fläschchen oder Einmalspritzen. Hier wird ein Probenahmeweg nur einmal benutzt und dann stillgelegt. Diese Methode erfordert deshalb räumlich große Konstruktionen und einen häufigen Wechsel der Gefäßvorlagen, wobei zusätzlich viel Einwegmaterial, z. B. Spritzen, anfällt.
- Thermische Desinfektion der Schnittstelle, z. B. mittels Dampf. Diese Methode erfordert druckfeste Verbindungen wie beispielsweise eine entsprechende Verrohrung sowie einen nicht unerheblichen Zeitaufwand für die Abkühlung und ist damit insbesondere für Laborreaktoren wenig geeignet.
- Verwendung von Membranen, die für Bakterien unpassierbar sind. Diese Methode wird vor allem für die Analysenmethode "FIA" (Flow injection analysis) angewandt. Bei dieser Methode ist aber nur eine zellfreie Probenahme möglich.

Allgemein gilt, daß die genannten Methoden bis auf das FIA-System wegen der notwendigen manuellen Bedienung keine Möglichkeit zur Automatisierung bieten und somit nicht universell einsetzbar sind.

Kommerziell erhältliche Systeme werden beispielsweise in Biotech-Forum 6, 274-288 (1989)beschrieben sowie von der Firma Bioengineering, CH 8636 Wald, vertrieben.

Der vorliegenden Erfindung liegt daher die Aufgabe zugrunde ein Probenahmesystem aufzufinden, welches folgende Mindestanforderungen erfüllt:
- kompakte Bauweise, einfacher Aufbau
- universell anwendbar für sterile Probenziehung
- sichere Betriebsweise
- keine Beeinflussung des Probenmaterials durch chemische Agenzien
- einfache Handhabung und ständige Zugriffsmöglichkeit
- kein Anfall von Verbrauchsmaterial wie Desinfektionsmittel, Spritzen etc.
- keine Limitierung in der Probenahmehäufigkeit
- vollautomatisierbares System, z.B. Anschlußmöglichkeit an Autosampler für Online-Analytik

Erfindungsgemäß wurde die Aufgabe dadurch gelöst, daß beim erfindungsgemäßen Probenahmegerät die Trennstelle zur mikrobiologisch belasteten Umgebung als steriler definierter Schutzgasstrom, welcher vorzugsweise aus steriler Luft besteht, nach dem "Laminar-Air-Flow-Prinzip" in der Weise ausgeführt ist, daß ein laminarer Schutzgasstrom (siehe beispielsweise Kohlbausch in Praktische Physik, Seite 138, Band 1, Teubner Verlag, Stuttgart 1968) zweckmäßigerweise von oben durch das Probenahmegerät geleitet wird, welches mindestens aus einer, vorzugsweise jedoch aus zwei konzentrisch angeordneten Röhren besteht, wobei der Zwischenraum zwischen der äußeren und inneren Röhre die Schutzhaube für die Sterilbegasung darstellt und die Verbindungsstelle der inneren Röhre (11) mit dem Proberöhrchen (10) so erfolgt, daß dies innerhalb bis knapp unterhalb der Gehäuseglocke (7) fest oder ohne feste Verbindung und für den automatischen Betrieb knapp unter der Gehäuseglocke (7) ohne feste Verbindung erfolgt.

Bei einem bevorzugten Probenahmegerät besteht das Probenahmegehäuse (7) vorzugsweise jedoch aus zwei konzentrisch angeordneten, oben miteinander fest verbundenen Röhren, vorzugsweise aus Glas. Durch die gemeinsame Mittelpunktslinie dieses Doppelgehäuses wird ein dünnes Rohr (11), vorzugsweise ebenfalls aus Glas, für den Transport der Probenflüssigkeit geführt. Die gesamte Gehäuseglocke kann über die dargestellten Luftfilteröffnungen (9) permanent mit einem turbulenzfreien und sterilfiltriertem Schutzgas oder Gas, vorzugsweise mit sterilfiltrierter Luft, versorgt werden, wobei vorzugsweise ein derartiger laminarer Gasstrom durch Durchleiten des Gases durch eine permeable Membran (8), welche oberhalb des Endes des inneren Rohrs (11) angeordnet ist, erzeugt wird und am unteren offenen Gehäuseende wieder ausströmt. Dieser Gasstrom verdrängt ab einer bestimmten Strömungsgeschwindigkeit die mikrobiologisch belastete Umgebungsluft und stellt per se einen Schutz der Probenaustrittsstelle vor Rückkontamination dar. Die Strömungsgeschwindigkeit liegt hierbei zweckmäßigerweise in einem Bereich von 0,1 bis 2 m/sec., vorzugsweise jedoch zwischen 0,4 und 0,6 m/sec., wie sie beispielsweise auch bei Sicherheitswerkbänken zur Erzeugung eines laminaren, wirbelungsarmen Luftstroms ebenfalls zur Anwendung kommt.

Der Vorgang der Probenahme selbst, also das Abfüllen der sterilen Flüssigkeit, beinhaltet folgende Möglichkeiten, was die überaus flexiblen Nutzungsmöglichkeiten der Vorrichtung belegt:

### (a) Fester Anschluß des Probengefäßes von Hand:

Das sterile Proberöhrchen wird hierzu von unten in die Gehäuseglocke (7) eingeführt und mit dem passenden Gegengewinde oder Schnellverschluß, der sich an der inneren Gehäuseröhre befindet, verbunden. Die Entlüftung des zu befüllenden Gefäßes erfolgt in diesem Falle über den Luftfilter (9.1).

### (b) Abfüllen der Probe von Hand ohne festen Anschluß:

Der obere Rand des Probengefäßes wird hierzu innerhalb der Gehäuseglocke (7) direkt unter das innere Rohr (11), also unterhalb den Probenauslauf, gehalten.

### (c) Automatisches Befüllen von Probenröhrchen ohne festen Anschluß:

Bei dieser Betriebsart werden mehrere Probengefäße, z.B. in ein rotierendes Probenkarussell gestellt (Autosampler) und automatisch zweckmäßigerweise direkt unterhalb der Gehäuseglocke (7) positioniert.

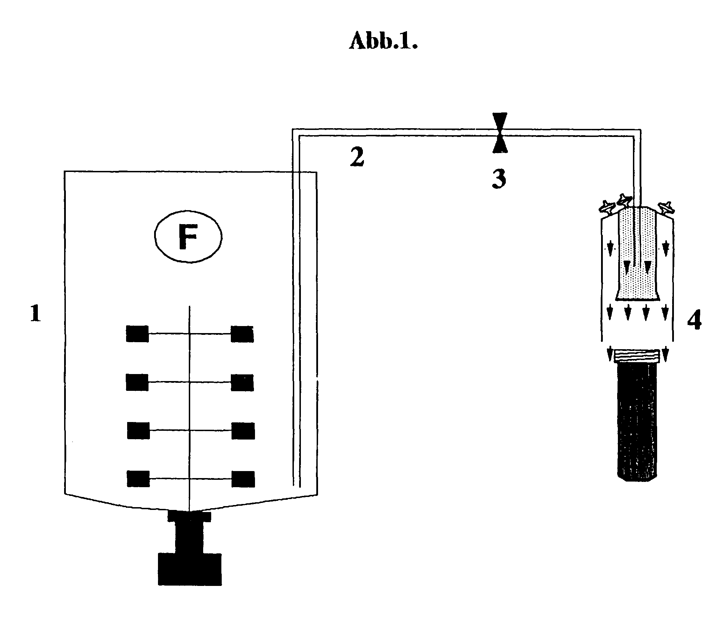

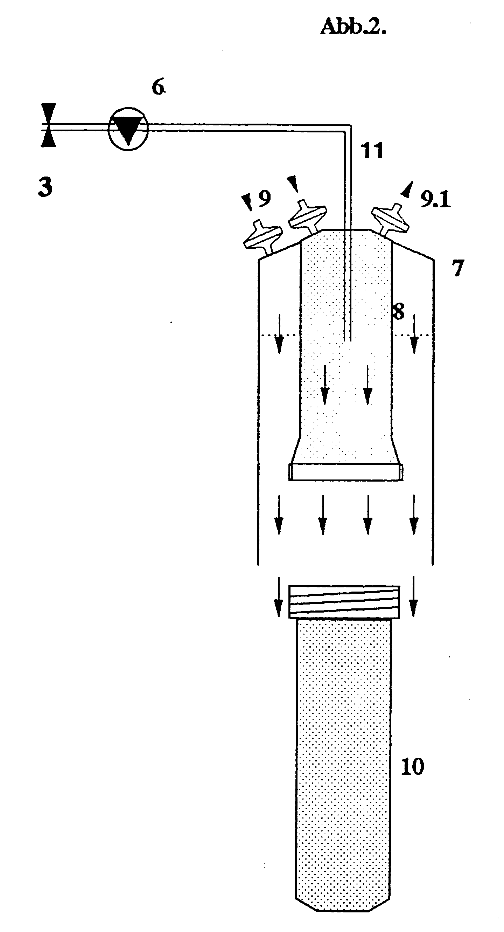

Die Abbildungen 1 und 2 beschreiben eine bevorzugte Ausführungsform des erfindungsgemäßen Probenahmegerätes:

**Abb.1** beschreibt die schematische Gesamtfunktion des Gerätes in Zusammenhang mit dem Bioreaktor.

### a) Sterilisation des Gerätes:

Das Probenahmegerät wird über eine geeignete Verbindung (3) und einem Schlauch, vorzugsweise aus Silikon, an den Ausgang des Probenahme-Tauchrohrs des Fermenters (1) angeschlossen und zusammen mit dem Fermenter autoklaviert. Dazu muß die Haube (7) am unteren Ende durch einen geeigneten Deckel verschlossen werden. Die Schnittstelle (3) ist hierbei zweckmäßigerweise eine handelsübliche Schnellkupplung für steriltechnische Verbindungen, z. B. eine der Firma Swagelock, USA), welche erforderlichenfalls einen Austausch oder nachträglichen Anbau des sterilisierten Gerätes erlaubt.

### b) Betriebsweise:

Nach der Sterilisation wird an die eine oder mehrere, vorzugsweise jedoch an 2 Schutzgaseintrittsöffnungen (9), welche vorzugsweise Luftfilter (z. B. Acrodisc, 0,2 µm Porenweite, Fa. Gelman) darstellen, ein inertes Schutzgas, vorzugsweise sterile Druckluft, wie sie in jedem Labor zur Verfügung steht, angelegt. Gleichzeig oder vorteilhafter vor der Entfernung des für die Sterilisation verwendeten Deckels wird der Vordruck des Gases derart eingestellt, daß eine Gasgeschwindigkeit von ca. 0,5 m/s erreicht wird. Dieser Aufbau bleibt während der gesamten Prozeßdauer erhalten und erlaubt einen ständigen Zugang zum Fermenterinhalt.

### Abb.2.stellt die Probenahmevorrichtung in ihrem Aufbau dar:

Das Gehäuse (7) besteht aus 2 konzentrisch angeordneten Röhren, welche vorzugsweise aus hitzebeständigem Glas gefertigt sind, wobei der Zwischenraum zwischen der äußeren und inneren Röhre die Schutzhaube für die Sterilbegasung darstellt. Die äußere Abmessung des Gehäuses beträgt bei dieser speziellen Beschreibung 10 cm Länge und 4 cm Durchmesser; es kann aber auch andere Dimensionen annehmen. In den inneren Gehäuseteil, welcher erforderlichenfalls mit einem oder mehreren Entlüftungsventilen (9.1) ausgestattet sein kann, wobei diese zweckmäßigerweise baugleich mit den Schutzgaseintrittsöffnungen sind, wird ein Rohr (11), z. B. ein Glasrohr, eingeführt, welches über eine Verbindung, z. B. einer Silikonschlauchverbindung, und über eine Schnellkupplung (5) mit dem Bioreaktor (1) verbunden wird. Eine Peristaltikpumpe (6), die zweckmäßigerweise zwischen der Schnellkuplung (5) und dem Probenahmegerät liegt, befördert das gewünschte Probenvolumen direkt in ein geeignetes Probengefäß (10). Die Zellkulturflüssigkeit wird dabei durch einen Gasstrom, der durch die oben beschriebenen Luftfilter (9) geführt wird, vor Fremdkontamination geschützt. Hierbei ist es von Vorteil, wenn eine permeable Membran (8), z.B. aus Polyvinyldifluorid oder eine geeignete Glasfritte, im Zwischenraum zwischen der äußeren und inneren Röhre der Schutzhaube (7) angebracht ist, um eine gleichmäßige Gasverteilung und einen laminaren Gasstrom zu gewährleisten. Durch die austretende laminare Strömung wird die Umgebungsluft verdrängt und eine Infektion der Probenflüssigkeit sicher verhindert.

Überraschenderweise reicht ein geringes Gehäusevolumen der Schutzhaube (7) zur Gewährleistung des sicheren Betriebes des Gerätes aus. Am beschriebenen Beispiel beträgt das äußere Gehäusevolumen nur ca. 110 cm³. Dies macht eine kompakte Bauweise der Vorrichtung möglich.

Bei manueller Probenahme wird ein vorsterilisiertes Probengefäß (10), idealerweise ein 15 ml-Röhrchen beispielsweise der Fa. Falcon auf den inneren Gehäusering des inneren Rohres aufgeschraubt, wobei auch andere Verbindungsarten wie Schnappverschlüsse in Betracht kommen, oder einfach unter das Glasrohr (11) zweckmäßigerweise innerhalb der Haube (7) gehalten. Bei einer festen Verbindung erfolgt die Entlüftung vorzugsweise über die zweckmäßigerweise baugleiche Filtereinheit (9.1.)

Bei automatisierter Probenahme wird das Probengefäß (10) in der dargestellten Weise positioniert und automatisch über Ansteuerung der Pumpe (6) befüllt. Hierbei ist das Probengefäß so unterhalb der Haube (7) in einem Abstand von 1 bis 50 mm, vorzugsweise jedoch 5 bis 20 mm angebracht, daß eine automatische Befüllung des Proberöhrchen möglich ist.

Vor jedem Meßzyklus muß in jedem Fall, um eine repräsentative Probe zu erhalten, ein bestimmtes Vorlaufvolumen, das dem Inhalt des Silikonschlauches bis zum Fermenter entspricht, verworfen werden.

Erfindungsgemäß ist das neuartige Probenahmegerät somit durch folgende Merkmale charakterisiert:
- es weist keine mechanisch bewegten Teile auf
- es verwendet vorzugsweise keimfrei filtrierte Druckluft oder ein anderes Gas als einziges Medium für den sterilen Betrieb, nachdem das Gerät zuvor thermisch inaktiviert wurde
- eine ständige definierte Gasströmung unterhalb der beschriebenen Glocke ermöglicht eine offene Handhabung des vorsterilisierten Probengefäßes, d. heißt, ein direkter Kontakt zum Probenahmegerät selbst ist nicht zwingend erforderlich
- ein Abfüllen von Proben ist jederzeit ohne Vorbereitung und ohne Zeitverlust möglich

## Patentansprüche

1. Probenahmesystem für die Bioprozeßanalytik, dadurch gekennzeichnet, daß die Trennstelle zur mikrobiologisch belasteten Umgebung als steriler Schutzgasstrom nach dem "Laminar-Air-Flow-Prinzip" in der Weise ausgeführt ist, daß ein laminarer Schutzgasstrom von oben durch das Probenahmegerät geleitet wird, welches aus 2 konzentrisch angeordneten Röhren besteht, wobei der Zwischenraum zwischen der äußeren und inneren Röhre die Schutzhaube für die Sterilbegasung darstellt, und die Verbindungsstelle der inneren Röhre (11) mit dem Proberöhrchen (10) so erfolgt, daß dies innerhalb bis knapp unterhalb der Gehäuseglocke (7) fest oder ohne feste Verbindung und für den automatischen Betrieb knapp unter der Gehäuseglocke (7) ohne feste Verbindung erfolgt.

2. Probenahmesystem gemäß Anspruch 1, dadurch gekennzeichnet, daß in die innere Röhre ein dünnes Rohr (11) für den Transport der Probenflüßigkeit eingeführt wird, welches über eine Verbindung und über eine Schnellkupplung (5) mit dem Bioreaktor (1) verbunden ist.

3. Probenahmesystem gemäß den Ansprüchen 1 und 2, dadurch gekennzeichnet, daß zwischen der Schnellkuplung (5) und dem Probenahmegerät eine Peristaltikpumpe (6) liegt.

4. Probenahmesystem gemäß den Ansprüchen 1 bis 3, dadurch gekennzeichnet, daß eine permeable Membran (8) im Zwischenraum zwischen der äußeren und inneren Röhre der Schutzhaube (7) angebracht ist.

## Claims

1. A sampling system for bioprocessing analysis, characterised in that the interface with the microbiologically contaminated environment is constructed as a sterile protective gas current on the laminar air-flow principle, in such a way that a laminar flow of protective gas is passed from above through the sampling device which consists of two concentrically mounted tubes, the space between the outer tube and the inner tube defining the protective hood for sterile gas flow and the connection site of the inner tube (11) to the sample tube (10) being such that it occurs inside to just below the housing bell (7), fixedly or with no fixed connection, and, for automatic operation, it occurs just below the housing bell (7) with no fixed connection.

2. A sampling system according to claim 1, characterised in that a thin tube (11) for transporting the sample fluid, which is connected to the bioreactor (1) via a connection and a quick-release coupling (5), is introduced into the inner tube.

3. A sampling system according to claims 1 and 2, characterised in that there is a peristaltic pump (6) between the quick-release coupling (5) and the sampling device.

4. A sampling system according to claims 1 to 3, characterised in that a permeable membrane (8) is arranged in the space between the outer and inner tubes of the protective hood (7).

## Revendications

1. Système de prélèvement d'échantillons pour l'analyse de processus biologiques, caractérisé en ce que le site de séparation avec l'environnement microbiologiquement chargé est réalisé sous forme de courant de gaz protecteur stérile selon le "principe Laminar Air-Flow" de telle manière qu'un courant de gaz protecteur laminaire est introduit d'en haut dans l'appareil de prélèvement d'échantillons qui consiste en deux tubes disposés concentriquement, l'espace entre le tube extérieur et le tube intérieur constituant la calotte de protection pour l'introduction de gaz stérile et le point de jonction du tube intérieur (11) avec le petit tube à échantillon (10) est réalisé de telle manière qu'il se produit à l'intérieur de jusqu'à juste au-dessous de la cloche d'enveloppe (7) de manière fixe ou sans liaison fixe et pour l'exploitation automatique juste sous la cloche d'enveloppe (7) sans liaison fixe.

2. Système de prélèvement d'échantillons selon la revendication 1, caractérisé en ce qu'à l'intérieur du tube intérieur est introduit un tube mince (11) pour le transport du liquide échantillon qui est relié au bioréacteur (1) par une liaison ou par un raccord rapide (5).

3. Système de prélèvement d'échantillons selon les revendications 1 et 2, caractérisé en ce qu'une pompe péristaltique (6) est située entre le raccord rapide (5) et l'appareil de prélèvement d'échantillons.

4. Système de prélèvement d'échantillons selon les revendications 1 à 3, caractérisé en ce qu'une membrane perméable (9) est disposée dans l'espace entre le tube extérieur et le tube intérieur de la calotte de protection (7).
